# EUROPÄISCHE PATENTANMELDUNG

(11) **EP 2 184 347 A2**
(43) Veröffentlichungstag der Anmeldung: **12.05.2010**
(21) Anmeldenummer: 09175335.0
(22) Anmeldetag: 06.11.2009
(51) Int. Cl.: C12M 3/00

(54) **Neue Komponenten und neues Bioreaktorsystem für die Kultur und mechanische Stimulation von biologischem Material**

(30) Priorität: 07.11.2008 DE 102008056951; 06.11.2008 EP 08168534; 07.11.2008 EP 08168650
(71) Anmelder: Duda, Georg N., 12209 Berlin (DE); Petersen, Ansgar, 12045 Berlin (DE)
(72) Erfinder: Duda, Georg N., 12209 Berlin (DE); Petersen, Ansgar, 12045 Berlin (DE)
(74) Vertreter: Gulde Hengelhaupt Ziebig & Schneider

(57) **Zusammenfassung**

Bioreaktor (50) zur Kultur und mechanischen Stimulation von biologischem Material umfassend eine Bioreaktorkammer, eine Zellkultureinheit (52) und eine Einheit (53) zur Ausübung mechanischer Stimuli auf das zu kultivierende biologische Material, dadurch gekennzeichnet, dass die Einheit (53) zur Ausübung mechanischer Stimuli mindestens zwei unabhängige, regel- und/oder steuerbare Antriebe aufweist, wobei ein erster Antrieb ein Piezoantrieb (71) ist, der eine Erzeugung mechanischer Stimuli erlaubt, und ein zweiter Antrieb ein Linearantrieb (61) ist, der eine Regulation der Position des biologischen Materials in der Bioreaktorkammer erlaubt.

## Beschreibung

Bioreaktoren werden auf dem Gebiet des sogenannten "tissue engineering" vielfältig eingesetzt, beispielsweise um Gewebe für eine spätere Transplantation in den menschlichen Körper zu kultivieren und zu funktionalisieren. In diesem Zusammenhang ist eine große Zahl unterschiedlicher Systeme bekannt, von denen ein Großteil der Herstellung von Knorpeltransplantaten dient (Donkelaar et al., Review on Patents for Mechanical Stimulation of Articular Cartilage Tissue Engineering. Recent Patents on Biomedical Engineering 2008, 1, 1-12).

Diese Systeme haben gemeinsam, dass neben der Schaffung geeigneter Zellkulturbedingungen (Sauerstoff, pH-Wert, Nährstoffe, Temperatur etc.) zusätzlich ein mechanischer Stimulus auf die Zellen appliziert wird. Mechanische Kräfte, die auf die Zellen wirken, sind eine Voraussetzung für die Herstellung von funktionalisierten "tissue engineering"-Konstrukten, wie z.B. künstlichem Knorpel, die dazu bestimmt sind Lasten im menschlichen Körper zu tragen.

Übliche Ansätze für eine mechanische Stimulation sind fluss-induzierte Kräfte (z. B. in Bioreaktoren mit Behältern mit rotierenden Wänden) (siehe z.B. in "An Overview on Bioreactor Design, Prototyping and Process Control for Reproducible Three-Dimensional Tissue Culture", in: Drug Testing In Vitro: Breakthroughs and Trends in Cell Culture Technology, Chapter 2, Edited by Uwe Marx and Volker Sandig, WILEY-VCH Verlag GmbH & Co. KGaA, Weinheim, 2007, ISBN: 978-3-527-31488-1), die Verwendung von hydrostatischem Druck (Hansen, U., Schünke, M., Domm, C., loannidis, N., Hassenpflug, J., Gehrke, T., Kurz, B., Combination of reduced oxygen tension and intermittent hydrostatic pressure: a useful tool in articular cartilage tissue engineering. Journal of Biomechanics, 34 (2001) 941-949) und direkte mechanische Kraft-Aufbringung (axiale Kompression, Dehnung, Biegung) (siehe z.B. in Seidel, J.O., Pei, M., Gray, M.L., Langer, R., Freed, L.E., and Vunjak-Novakovic, G., Longterm culture of tissue engineered cartilage in a perfused chamber with mechanical stimulation. Biorheology 41 (2004): 445-458).

Auf der anderen Seite ist die Applikation mechanischer Kräfte auf Zell-Konstrukte ein Weg, um solche Prozesse zu untersuchen, die während eines Heilungsvorganges von spezifischen Defekten im menschlichen Körper ablaufen. Ein Beispiel dafür ist die Knochenregeneration nach einer Fraktur oder einer Osteotomie. In 10-20% der klinischen Fälle werden Komplikationen im Heilungsprozess in Form einer verzögerten Heilung oder sogar dem Ausbleiben der Knochenüberbrückung beobachtet (Audige, L., Griffin, D., Bhandari, M., Kellam, J., Ruedi, T. P. (2005): Path analysis of factors for delayed healing and nonunion in 416 operatively treated tibial shaft fractures. Clin Orthop Relat Res, 438: 221-32).

Untersuchungen in einem Bioreaktor-System haben den Einfluss mechanischer Belastung auf die Expression bestimmter Gene und damit auf die Zell-Differenzierung demonstriert (Matziolis, G., Tuischer, J., Kasper, G., Thompson, M., Bartmeyer, B., Krocker, D., Perka, C., Duda, G., Simulation of Cell Differentiation in Fracture Healing: Mechanically Loaded Composite Scaffolds in a Novel Bioreactor System. Tissue Engineering, 12(1) (2006): 201-208; und Kasper. G., Glaeser, J.D., Geissler, S., Ode, A., Tuischer, J., Matziolis, G., Perka, C., Duda G.N., Matrix metalloprotease activity is an essential link between mechanical stimulus and mesenchymal stem cell behavior. Stem Cells, 25 (2007): 1985-1 994). Diese Untersuchungen haben gleichzeitig ergeben, dass es möglich ist, das grundlegende Verhalten eines heilenden Fraktur-Kallus in einem in vitro System zu simulieren.

Dabei ist es besonders erstrebenswert, das biologische Material über einen möglichst langen Zeitraum definierten, kontrollierbaren Bedingungen aussetzen zu können, während das biologische Material in seiner Kulturumgebung verbleibt und nicht mit Materialien aus der Umgebung in Kontakt gelangt.

Im Bioreaktor nach Seidel at al. wird der mechanische Stimulus auf das biologische Material durch das Eindrücken einer Silikonmembran mittels eines externen Stempels erreicht. Diese Herangehensweise hat den Nachteil, dass das Medium-gefüllte Lumen der Reaktorkammer des Bioreaktors dabei beeinträchtigt wird und dadurch undefinierte Volumen- und Druckveränderungen in der Reaktorkammer entstehen.

In einem in WO 05/040332 beschriebenen Bioreaktorsystem wird der mechanische Stimulus durch einen magnetischen Stempel appliziert, der über einen außerhalb des Reaktorraumes angeordneten Magneten gesteuert werden kann. Dieses System hat das Problem, dass solche Magnetsteuerungen limitiert sind in der Möglichkeit den mechanischen Stimulus sowohl bzgl. der absoluten Kraft als auch bzgl. der Bewegungsform ausreichend genau zu steuern. Die applizierbare Frequenz ist dabei auf wenige Hertz begrenzt. Außerdem erlaubt dieses System lediglich die Applikation eines mechanischen Stimulus von einer Seite des biologischen Materials. Zwei solcher magnetischer Stempel mit unterschiedlicher Ausrichtung lassen sich nicht ohne weiteres von Außen ausreichend steuern oder regeln.

Ein weiteres Problem bei der mechanischen Stimulation von biologischem Material über einen längeren Zeitraum in einem Bioreaktor ist, dass es über die Zeit zu irreversiblen Deformationen des biologischen Material kommen kann und dadurch am deformierten biologischen Material nicht mehr die selben mechanischen Kräfte wirken wie zu Anfang der Behandlung.

Aufgabe der vorliegenden Erfindung ist es, einen oder mehrere Nachteile des Standes der Technik zu vermindern oder zu überwinden. Insbesondere ist es eine Aufgabe der Erfindung Mittel bereitzustellen, die es erlauben biologisches Material über einen längeren Zeitraum definierten mechanischen Stimuli auszusetzen.

Zur Lösung dieser Aufgabe stellt die vorliegende Erfindung einen Bioreaktor zur Kultur und mechanischen Stimulation von biologischem Material bereit. Der erfindungsgemäße Bioreaktor umfasst eine Bioreaktorkammer, eine Zellkultureinheit und eine Einheit zur Ausübung mechanischer Stimuli auf das zu kultivierende biologische Material, wobei die Einheit zur Ausübung mechanischer Stimuli mindestens zwei unabhängige, regel- und/oder steuerbare Antriebe aufweist, wobei ein erster Antrieb ein Piezoantrieb ist, der eine Erzeugung mechanischer Stimuli erlaubt, und ein zweiter Antrieb ein Linearantrieb ist, der eine Regulation der Position des biologischen Materials in der Bioreaktorkammer erlaubt.

Durch die Verwendung dieser beiden unabhängigen Antrieben für die mechanische Stimulation lassen sich nun verschiedenste Kraftmuster darstellen. Durch die Verwendung eines Piezoantriebs ist nun auch die Bereitstellung mechanischer Stimuli in höheren Frequenzen ermöglicht. Dadurch, dass nun zwei unabhängige Antriebe vorgesehen sind, kann beispielsweise ein Antrieb zur Einleitung der gewünschten mechanischen Stimuli verwendet werden, während der andere Antrieb z.B. zur Regulation der Position des biologischen Materials in der Reaktorkammer eingesetzt werden kann. Durch geeignete Regulation der Probenposition lässt sich beispielsweise der Effekt einer irreversiblen Probendeformation über die Zeit ausgleichen und sicherstellen, dass am biologischen Material in der Reaktorkammer konstante, von dem anderen Antrieb bereitgestellte, definierte Kraftmuster wirken.

Die Verwendung dieser beiden unabhängigen Antriebe ermöglicht eine kompakte Bauform, sodass mehrere mit diesen Antrieben versehene Bioreaktoreinheiten (≥8 Stück) in einem für die Zellkultur üblichen Inkubator Platz finden.

Im erfindungsgemäßen Bioreaktor kann ein Piezoantrieb eingesetzt werden, der eine Erzeugung mechanischer Stimuli mit einer maximalen Frequenz von ≥ 5 Hz erlaubt.

Der erfindungsgemäße Bioreaktor kann zusätzlich einen Kraftmesser aufweisen, der derart angeordnet und ausgestaltet ist, dass darüber die tatsächlich auf das biologische Material einwirkenden Kräfte bestimmbar sind.

Der Bioreaktor kann zusätzlich eine Steuer- und/oder Regelungseinheit aufweisen, die derart angeordnet und ausgestaltet ist, dass die Position des biologischen Materials in Abhängigkeit von den am Kraftmesser gemessenen Werten regulierbar ist.

Im erfindungsgemäßen Bioreaktor kann die Zellkultureinheit eine Mikropumpe und/oder ein Kulturmediumreservoir mit integrierter Gasaustauschmembran umfassen.

Der erfindungsgemäße Bioreaktor kann eine erfindungsgemäße Bioreaktorkammer und/oder eine oder mehrere erfindungsgemäße Dichtungen aufweisen.

Die vorliegende Erfindung bezieht sich auch auf eine Dichtung zur Abdichtung einer Bioreaktorkammer. Diese Dichtung umfasst:
a) ein Formelement, welches ausgebildet ist zur flüssigkeits- und/oder gasdichten Abdichtung eines Kontaktbereichs der Dichtung mit einer Oberfläche einer Bioreaktorkammer; und
b) ein Dichtungselement, wobei das Dichtungselement eine Rollmembran aufweist, die einen Aufnahmebereich zur flüssigkeits- und/oder gasdichten Aufnahme einer Kolbenstange und einen Rollbereich umfasst.

Wird in die Aufnahme des Dichtungselementes der Dichtung eine Kolbenstange aufgenommen, so kann diese Kolbenstange in längsaxialer Richtung derart bewegt werden, dass der Rollbereich nach innen bzw. außen rollt oder gestülpt wird, während Aufnahme und Kolbenstange flüssigkeits- und/oder gasdicht verbunden bleiben und im Wesentlichen keine Relativbewegung zueinander zulassen. Die Dichtung bleibt während dieser Bewegung flüssigkeits- und/oder gasdicht, es ist keine besondere Abdichtung von gleitenden Teilen notwendig. Es treten bei der Bewegung auch keine Teile oder Oberflächen in den Reaktorraum ein, die zu Beginn oder während der Bewegung ggf. außerhalb des Reaktorraumes angeordnet waren, so dass der Reaktorraum während der Bewegung der Kolbenstange geschlossen, dicht und dadurch keimfrei und/oder steril verbleibt. Die erfindungsgemäße Dichtung erlaubt damit die Übertragung einer längsaxialen Bewegung aus der Umgebung in das Lumen einer Bioreaktorkammer mittels einer in der Aufnahme der Dichtung gelagerten Kolbenstange über eine festgelegte Wegstrecke, die von der gewählten Dimensionierung des Rollbereichs der Rollmembran der Dichtung bestimmt wird, ohne dass der Rest des Dichtungselementes einer nennenswerten Verformung unterzogen wird. Die Verwendung einer solchen Dichtung erlaubt es also, mechanische Stimuli auf ein in einer Bioreaktorkammer angeordnetes biologisches Material zu übertragen, wobei das Lumen der Bioreaktorkammer dabei nur minimal beeinflusst wird.

Die erfindungsgemäße Dichtung kann sich dadurch auszeichnen, dass im Aufnahmebereich eine Kolbenstange derart kraftschlüssig, stoffschlüssig und/oder formschlüssig aufnehmbar ist, dass eine Relativbewegung von Kolbenstange zum Aufnahmebereich im Wesentlichen unterbunden ist.

Der Rollbereich der Dichtung kann derart ausgestaltet sein, dass der Aufnahmebereich in längsaxialer Richtung über eine vorher festgelegte Strecke bewegbar ist, ohne dass es im Wesentlichen zu einer Verformung des Dichtungselementes außerhalb der Rollmembran kommt.

Die Dichtung oder Teile davon, wie beispielsweise das Formelement oder das Dichtungselement, können Silikon oder ein Silikon-haltiges Material aufweisen oder daraus bestehen.

Das Formelement der Dichtung kann eine Dichtkante und/oder eine Dichtfläche aufweisen und gegebenenfalls eine oder mehrere Dichtwülste umfassen.

Die vorliegende Erfindung bezieht sich auch auf eine Bioreaktorkammer zur Verwendung in einem Bioreaktor, umfassend einen Grundkörper und mindestens eine erfindungsgemäße Dichtung, wobei:
- Grundkörper und Dichtung zusammen einen flüssigkeits- und/oder gasdichten Reaktorraum bilden;
- die Bioreaktorkammer wenigstens zwei Anschlüsse aufweist, über die dem Reaktorraum Flüssigkeit und/oder Gas zu- und/oder abführbar ist;
- im Reaktorraum ein Stempel angeordnet ist, der über eine, in der Aufnahme des Dichtungselements der Dichtung gelagerte Kolbenstange in längsaxialer Richtung bewegbar ist; und
- im Reaktorraum eine Vorrichtung zur Aufnahme von biologischem Material derart angeordnet ist, dass auf der Vorrichtung platziertes biologisches Material durch längsaxiale Bewegung des Stempels einer mechanischen Kraft aussetzbar ist.

In der erfindungsgemäßen Bioreaktorkammer können der oder die Stempel und/oder die Vorrichtung zur Aufnahme von biologischem Material derart ausgestaltet sein, dass platziertes biologisches Material während der Kultur von Kulturmedium umströmbar ist.

In der Bioreaktorkammer können der oder die Stempel und/oder die Vorrichtung zur Aufnahme von biologischem Material einen Bereich aufweisen, der während der Kultur mit dem biologischen Material direkt oder indirekt in Kontakt treten kann, wobei dieser Bereich von vier radial angeordneten, zentral miteinander verbundenen V-förmigen Auflageelementen gebildet wird und die Kontaktflächen der einzelnen V-förmigen Auflageelemente durch mit Kulturmedium durchströmbare Kanäle voneinander getrennt vorliegen.

Die Bioreaktorkammer kann zwei gegenüberliegend angeordnete, erfindungsgemäße Dichtungen aufweisen, wobei im Reaktorraum die Stempel der beiden Dichtungen sich gegenüberstehend angeordnet sind, wobei die Stempel unabhängig voneinander über die in der Aufnahme der jeweiligen Dichtung gelagerte Kolbenstange in längsaxialer Richtung bewegbar sind und wobei einer der beiden Stempel als Vorrichtung zur Aufnahme von biologischem Material dient.

In der Bioreaktorkammer können die beiden Stempel durch Führungsmittel aufeinander ausgerichtet vorliegen, so dass sich, bei längsaxialer Bewegung der Stempel aufeinander zu, die Stempel im Wesentlichen in der vorher festgelegten Ausrichtung aufeinander zu bewegen.

Im Folgenden wird der Gegenstand der Erfindung anhand von Ausführungsbeispielen näher erläutert. Die dazugehörigen Abbildungen zeigen:
- Fig. 1:: Schnittdarstellung einer ersten Ausführungsform einer erfindungsgemäßen Dichtung
- Fig. 2:: Schnittdarstellung einer zweiten Ausführungsform einer erfindungsgemäßen Dichtung
- Fig. 3:: Isometrische Darstellung einer erfindungsgemäßen Bioreaktorkammer
- Fig. 4:: Schnittdarstellung einer erfindungsgemäßen Bioreaktorkammer
- Fig. 5:: Schnittdarstellung einer erfindungsgemäßen Bioreaktorkammer mit Sichtfenster
- Fig. 6:: Isometrische Darstellung eines ersten Stempels
- Fig. 7:: Isometrische Darstellung eines zweiten Stempels
- Fig. 8:: Isometrische Darstellung eines erfindungsgemäßen Bioreaktors
- Fig. 9:: Schnittdarstellung eines erfindungsgemäßen Bioreaktors

### Dichtung:

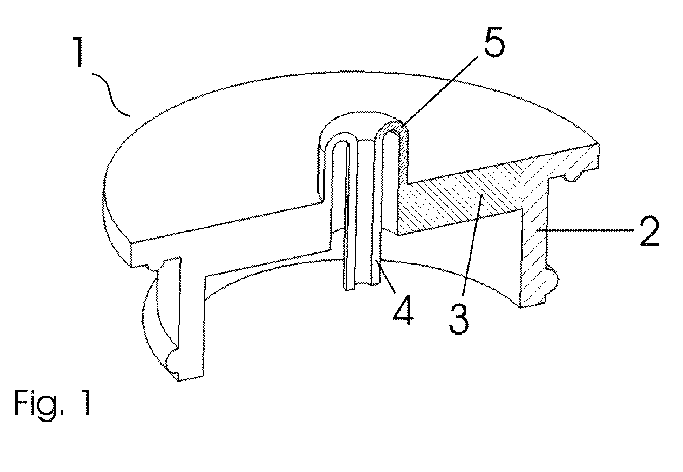

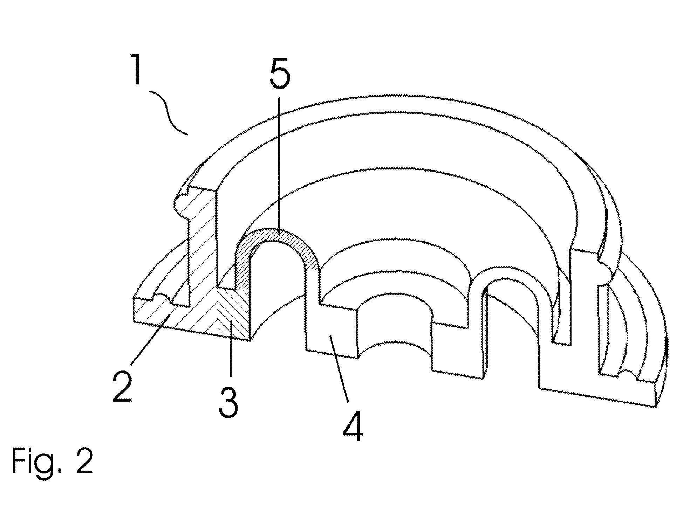

In Fig. 1 und 2 sind Ausführungsbeispiele der erfindungsgemäßen Dichtung gezeigt.

Dabei ist die erfindungsgemäße Dichtung 1 derart ausgestaltet, dass sie zur Abdichtung einer Bioreaktorkammer dienen kann. Unter einer Bioreaktorkammer wird dabei die Vorrichtung eines Bioreaktors verstanden, in der das entsprechende biologische Material während des Kultur- und/oder Versuchszeitraums angeordnet und definierten Bedingungen ausgesetzt werden kann. Unter biologischem Material wird dabei jedes Material verstanden, welches biologischen Ursprungs ist und bevorzugt über einen bestimmten Zeitraum kultivierbar ist. Das biologisches Material umfasst Viren, Zellen, Mischungen oder Zusammensetzungen von Zellen unterschiedlichen Typs, Zellverbände, Gewebe, Organe oder Teile davon sowie biologische Trägermaterialien für diese biologischen Komponenten, und sowie ganze Organismen, insbesondere Mikroorganismen wie Bakterien, Pilze und/oder Algen. Dabei ist das biologische Material nicht auf einen bestimmten Ursprung oder Spender begrenzt (z.B. humanen oder pflanzlichen Ursprungs).

Die erfindungsgemäße Dichtung 1 und die entsprechende Bioreaktorkammer sind derart aufeinander abgestimmt, dass die Dichtung 1 eine Öffnung der Bioreaktorkammer flüssigkeits- und/oder gasdicht von der Umgebung abschließen kann. Für die Zwecke der vorliegenden Erfindung wird unter "flüssigkeits- und/oder gasdicht" verstanden, dass während des Betriebs der Bioreaktorkammer im Wesentlichen keine für die Kultur erheblichen Flüssigkeiten und/oder Gase aus der Umgebung unkontrolliert in den Reaktorraum eintreten können. Dadurch wird einerseits gewährleistet, dass die Kultur unter konstanten, definierten Bedingungen durchgeführt werden kann und andererseits keine Keime oder andere störende Materialien eindringen können, so dass eine möglichst lange Kultur des biologischen Materials ermöglicht ist. Bevorzugt treten durch eine flüssigkeits- und/oder gasdichte Verbindung auch im Wesentlichen keine für die Kultur erheblichen Flüssigkeiten und/oder Gase aus dem Reaktorraum unkontrolliert in die Umwelt aus.

Die erfindungsgemäße Dichtung 1 umfasst ein Formelement 2 und ein Dichtungselement 3.

Das Formelement 2 ist derart ausgebildet, dass es eine flüssigkeits- und/oder gasdichte Abdichtung eines Kontaktbereichs der Dichtung mit einer Oberfläche einer Bioreaktorkammer erlaubt. Das Formelement 2 ist so geformt, dass es mit einer Oberfläche einer Bioreaktorkammer kontaktierbar ist, so dass eine Öffnung des Bioreaktors durch die Dichtung 1 flüssigkeits- und/oder gasdicht verschließbar ist. Dazu kann das Formelement 2 eine Dichtkante und/oder eine Dichtfläche aufweisen. Gegebenenfalls kann das Formelement 2 eine oder mehrere Dichtwülste aufweisen. In Fig. 1 und 2 ist das Formelement 2 beispielsweise als radial umlaufender, "L"-förmiger Steg ausgebildet, wobei das lange Ende des "L" eine Dichtfläche und das kurze Ende des "L" eine Dichtkante darstellt. Dabei weisen sowohl Dichtfläche als auch Dichtkante jeweils eine separate Dichtwulst auf.

Zum Erzielen der Dichtwirkung kann es notwendig sein, dass die Dichtung 1, insbesondere Teile des Formelements 2, durch weitere Mittel mit der Bioreaktorkammer verbunden oder daran fixiert werden. Solche Mittel können Schraubverbindungen umfassen, wobei beispielsweise eine Dichtkante des Formelementes 2 auf eine Oberfläche der Bioreaktorkammer gepresst wird, z.B. durch Aufschrauben eines Gewindeteiles auf ein dazu komplementäres Gewinde, welches an der Bioreaktorkammer vorgesehen ist.

Die Dichtung 1 weist darüber hinaus ein Dichtungselement 3 auf. Das Dichtungselement 3 dient dazu die Fläche der durch die Dichtung 1 zu verschließenden Öffnung einer Bioreaktorkammer ganz oder teilweise zu bedecken oder auszufüllen. Dabei können die Übergänge zwischen Formelement 2 und Dichtungselement 3 fließend sein und ein Bereich der Dichtung 1 kann beispielsweise sowohl als Formelement 2 als auch als Dichtungselement 3 dienen.

Das Dichtungselement 3 weist eine Rollmembran auf, die einen Aufnahmebereich 4 und einen Rollbereich 5 umfasst.

Der Aufnahmebereich 4 dient zur flüssigkeits- und/oder gasdichten Aufnahme einer Kolbenstange, so dass eine Relativbewegung zwischen Kolbenstange und Aufnahmebereich 4 im Wesentlichen nicht möglich ist. Bevorzugt kann der Aufnahmebereich 4 derart ausgestaltet sein, dass eine Kolbenstange kraftschlüssig, stoffschlüssig und/oder formschlüssig darin aufnehmbar ist, so dass eine Relativbewegung von Kolbenstange zum Aufnahmebereich 4 im Wesentlichen unterbunden ist. Dazu kann der Aufnahmebereich 4 der Dichtung 1 aufgrund von Dimensionierung und/oder von Materialeigenschaften der Materialien des Aufnahmebereichs 4 derart mit einer Kolbenstange in Kontakt stehen, dass eine Relativbewegung bei den zu erwartenden Kräften, die während der bestimmungsgemäßen Verwendung der Dichtung 1 auftreten können, im Wesentlichen unterbleibt. Die Aufnahme der Kolbenstange im Aufnahmebereich 4 der Dichtung 1 kann reversibel oder irreversibel erfolgen. Die Kolbenstange kann lediglich aufgrund der Spannkraft des Materials des Aufnahmebereichs 4 kraftschlüssig im Aufnahmebereich 4 aufnehmbar sein. Die Kolbenstange kann aber auch z.B. durch Verkleben oder Verschweißen stoffschlüssig mit dem Aufnahmebereich 4 verbindbar sein. Es ist auch möglich, dass die Kolbenstange und der Aufnahmebereich 4 formschlüssig, z.B. in einer Art "Nut-und-Feder-Prinzip", miteinander verbindbar ausgestaltet sind. Es sind auch Kombinationen verschiedener Aufnahmetechniken umfasst.

Der Rollbereich 5 verbindet den Aufnahmebereich 4 mit dem restlichen Dichtungselement 3 und stellt das zweite Funktionselement der Rollmembran dar. Der Rollbereich umfasst eine Membran aus flexiblem Material, die in Form eines "U" ausgestaltet ist, so dass zwei Lagen der Membran zueinander parallel geführt und an einem Ende miteinander verbunden sind. Wird nun der Aufnahmebereich 4, z.B. nach Aufnahme einer Kolbenstange durch eine auf die Kolbenstange ausgeübte Kraft, längsaxial bewegt, so "rollt" oder stülpt sich die Membran des Rollbereichs 5 entweder ab oder auf, je nach Bewegungsrichtung des Aufnahmebereichs 4, und erlaubt somit eine Bewegung des Aufnahmebereichs, ohne dass es im Wesentlichen zu einer Verformung des Dichtungselementes außerhalb der Rollmembran kommt. Damit wird eine Übertragung einer Bewegung durch die Dichtung 1 ermöglicht, ohne dass ein Gleiten von Teilen durch die Membran erforderlich ist und ohne dass weitere Teile des Dichtungselementes 3 in längsaxialer Richtung verformt werden. Eine Bioreaktorkammer mit einer solchen Dichtung 1 ist nicht nur flüssigkeits- und/oder gasdicht verschlossen, sondern es kann über eine im Aufnahmebereich 4 platzierte Kolbenstange auch ein mechanischer Stimulus in den Reaktorraum der Bioreaktorkammer übertragen werden, ohne das es zu einer nennenswerten Beeinträchtigung des Reaktorkammerlumens kommt.

Bevorzugt kann der Rollbereich 5 derart ausgestaltet sein, dass der Aufnahmebereich 4 in längsaxialer Richtung über eine vorher festgelegte Strecke bewegbar ist, ohne dass es im Wesentlichen zu einer Verformung des Dichtungselementes 3 außerhalb der Rollmembran kommt. Dabei kann über die Dimensionierung des Rollbereichs 5 der Rollmembran, die maximale Laufstrecke des Aufnahmebereichs 4 und damit der maximal erzielbare Aufwärts- und/oder Abwärtshub in der Bioreaktorkammer bestimmt und festgelegt werden.

In Fig. 1 ist eine Ausführungsform der erfindungsgemäßen Dichtung 1 für die Aufnahme einer Kolbenstange mit kleinem Durchmesser gezeigt, in Fig. 2 ist eine zweite Ausführungsform der erfindungsgemäßen Dichtung 1 für die Aufnahme einer Kolbenstange mit einem relativ größeren Durchmesser gezeigt. Je nach Ausgestaltung der Dichtung 1, kann die Rollmembran einen mehr oder weniger großen Anteil des Dichtungselementes 3 ausmachen. Es ist auch möglich, dass das Dichtungselement 3 im Wesentlichen aus der Rollmembran besteht.

Der Rollbereich 5 der Dichtung 1 ist dabei aus einem flexiblen Material geformt, welches ein mehrmaliges "Auf- und Abrollen" des Rollbereichs erlaubt. Bevorzugt ist der Rollbereich 5 aus dem selben Material geformt wie das Dichtungselement 3. Besonders bevorzugt ist die ganze Dichtung 1 aus dem selben Material geformt. Die Dichtung 1 kann einteilig hergestellt sein oder aus einer Mehrzahl von einzeln hergestellten Bestandteilen zusammengesetzt sein. Bevorzugt besteht die Dichtung 1 mit Formelement 2 und Dichtungselement 3 aus einem einzigen Formteil, dass aus dem selben Material hergestellt ist. Ein Vorteil einer einteiligen Dichtung 1 liegt darin, dass es keine Materialübergänge oder Verbindungen gibt, die ggf. besonders verschleißanfällig sein können oder unterschiedliche Beständigkeiten oder Lebensdauern aufweisen können. Bevorzugt weist die Dichtung 1, das Formelement 2 und/oder das Dichtungselement 3 Silikon oder ein Silikon-haltiges Material auf oder besteht daraus.

### Bioreaktorkammer:

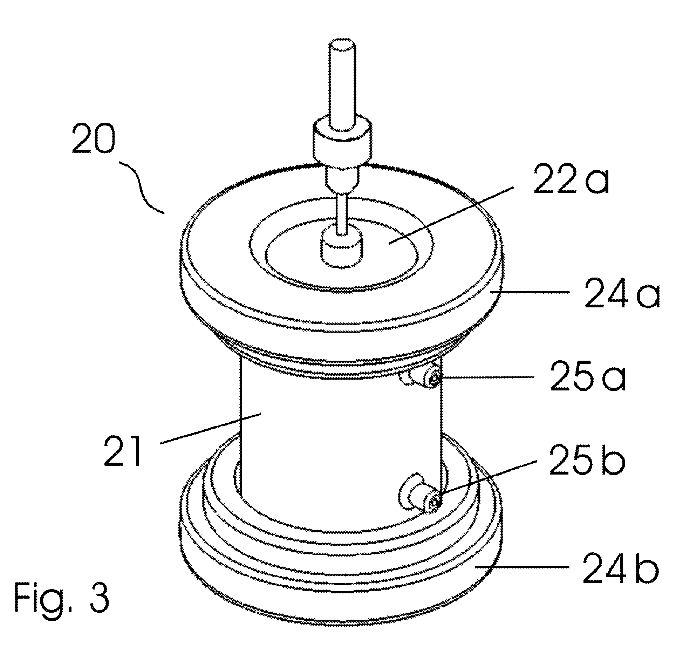

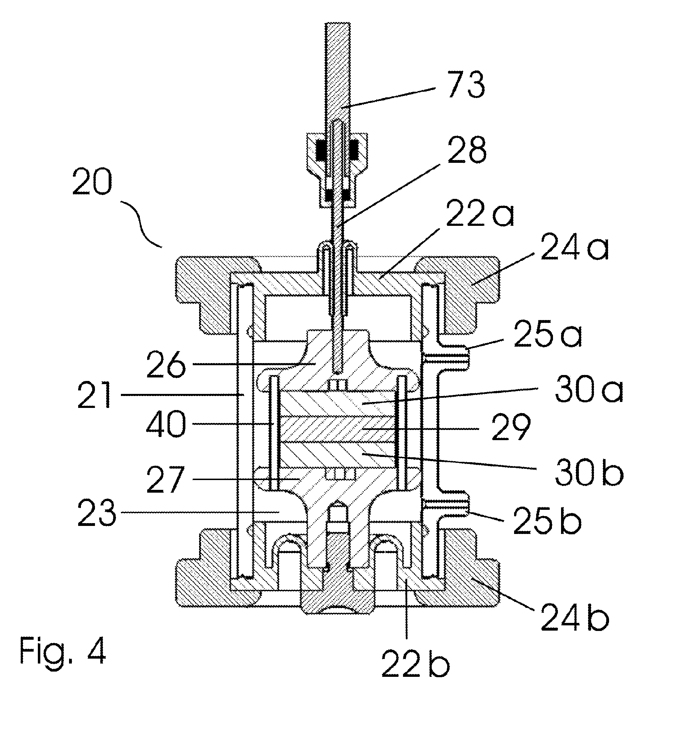

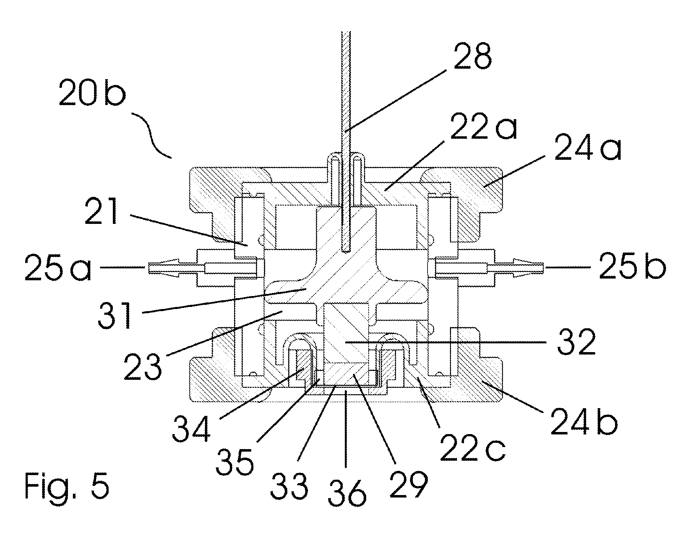

In Fig. 3 und Fig. 4 ist eine erfindungsgemäße Bioreaktorkammer in isometrischer bzw. in Schnittdarstellung gezeigt. In Fig. 5 ist eine erfindungsgemäße Bioreaktorkammer als Ausführung mit optischem Fenster in Schnittdarstellung dargestellt

Die erfindungsgemäße Bioreaktorkammer 20 dient zur Verwendung als Reaktionsraum in einem Bioreaktor. Die Bioreaktorkammer 20 umfasst einen Grundkörper 21 und zwei erfindungsgemäße Dichtungen 22a und 22b. Dabei kann die Dichtung 22a der Dichtung 1 aus Fig. 1 und die Dichtung 22b entspricht der Dichtung 1 aus Fig. 2 entsprechen. Der Grundkörper 21 und die Dichtungen 22a und 22b bilden zusammen den flüssigkeits- und/oder gasdichten Reaktorraum 23. Zu diesem Zweck sind die Dichtungen 22a und 22b mit den Gewindeschrauben 24a, respektive 24b am Grundkörper 21 fixiert. Der Reaktorraum 23 dient der Aufnahme und Kultur des biologischen Materials. Im Reaktorraum 23 kann das biologische Material sowohl definierten Kulturbedingungen als auch mechanischen Stimuli ausgesetzt werden. Der Grundkörper kann beispielsweise aus einen Material gefertigt sein, dass Glas, Polymethylmethacrylat (PMMA), Polyoxymethylen (POM), Polyetheretherketon (PEEK) und/oder Silikon enthält oder daraus besteht.

Die Bioreaktorkammer 20 weist mindestens zwei Anschlüsse 25a und 25b auf, über die dem Reaktorraum 23 Flüssigkeit und/oder Gas zu- und/oder abgeführt werden können. Über diese Anschlüsse 25a und 25b kann das biologische Material im Reaktorraum 23 beispielsweise mit dem notwendigen Kulturmedium versorgt werden. Dadurch, dass zwei unabhängige Anschlüsse 25a und 25b vorgesehen sind, ist es möglich im Reaktorraum 23 einen ständigen Flüssigkeits- und/oder Gasstrom zu erzeugen und ggf. das biologische Material 29 unter laminaren Strömungsbedingungen zu kultivieren. Da immer wieder neue Nährstoffe zugeführt und verbrauchtes Medium abgeführt werden können, ist der Zeitraum für die Kultur des biologischen Materials im Reaktorraum 23 nicht durch die Versorgungslage beschränkt und es sind grundsätzlich lange Kulturzeiträume von > 1 Woche möglich.

Im Reaktorraum 23 ist eine Vorrichtung zur Aufnahme von biologischem Material 27 angeordnet. Diese Vorrichtung 27 ist im Aufnahmebereich der Dichtung 22b durch Verplombung fixiert und ist damit in längsaxialer Richtung der Bioreaktorkammer 20 im Reaktorraum 23 bewegbar.

Des weiteren ist im Reaktorraum 23 ein Stempel 26 angeordnet. Der Stempel 26 ist über eine Kolbenstange 28 in längsaxialer Richtung im Reaktorraum 23 von außen bewegbar. Dabei ist die Kolbenstange 28 in der Aufnahme des Dichtungselements der Dichtung 22a flüssigkeits- und/oder gasdicht aufgenommen. Der Stempel 26 ist derart oberhalb der Vorrichtung 27 zur Aufnahme von biologischem Material platziert, so dass ein mechanischer Stimulus von außerhalb der Bioreaktorkammer 20 über den Stempel 26 und/oder die Vorrichtung 27 in den Reaktorraum 23 und auf das dort platzierte biologische Material 29 übertragbar ist.

Die Bioreaktorkammer 20 weist zwei Dichtungen 22a und 22b auf, wobei der Stempel 26 durch die Dichtung 22a hindurch von Außen bewegt werden kann, während die Vorrichtung zur Aufnahme von biologischem Material 27 durch die Dichtung 22b hindurch von Außen bewegt werden kann. Stempel 26 und Vorrichtung 27 sind direkt opponiert zueinander angeordnet, so dass das biologische Material 29 sowohl über den Stempel 26 als auch über die Vorrichtung 27 in seiner räumlichen Lage verändert werden und/oder einem mechanischen Stimulus z.B. durch eine periodische Kompression ausgesetzt werden kann. Damit kann das biologische Material 29 im Reaktorraum 23 unterschiedlichsten Kompressionen, mechanischen Stimuli und/oder Lageveränderungen ausgesetzt werden, sowie Kombinationen davon. Die mechanischen Kompressionen, Stimuli und/oder Lageveränderungen des biologischen Materials 29 können dabei aus zwei unterschiedlichen Richtungen übertragen oder vorgenommen werden.

Die vorliegende Erfindung umfasst allerdings auch solche Bioreaktorkammern, bei denen nur eine erfindungsgemäße Dichtung vorgesehen ist und ein mechanischer Reiz nur von einer Richtung in den Reaktorraum übertragbar ist.

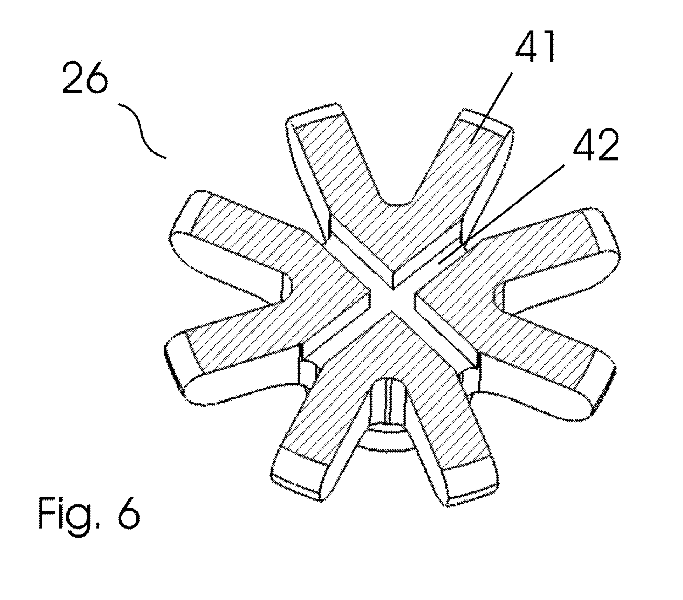

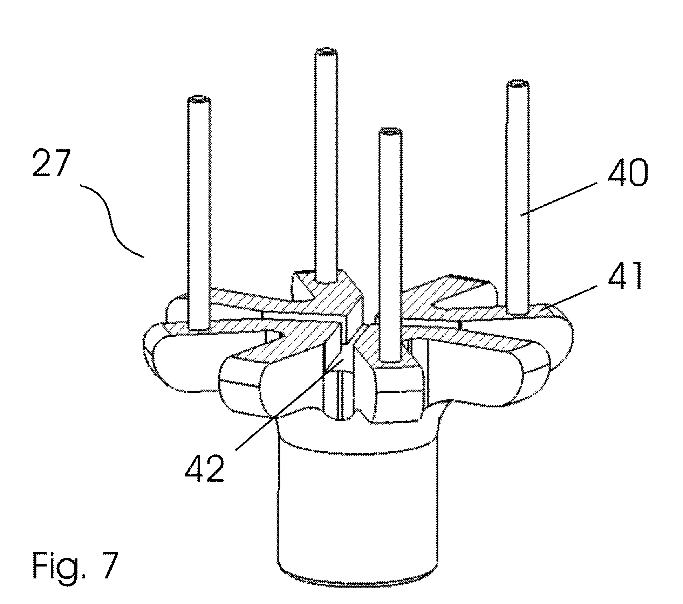

Bevorzugt sind der Stempel 26 und die Vorrichtung 27 derart ausgestaltet, dass das biologische Material während der Kultur und/oder der mechanischen Stimulation im Reaktorraum 23 im Wesentlichen von allen Seiten von Kulturmedium umströmbar ist. Dazu ist die Auflagefläche der Vorrichtung 27 und des Stempels 26, die mit dem biologischen Material kontaktierbar ist oder in Kontakt steht, möglichst klein, um einen Stoffaustausch zwischen dem Kulturmedium und dem biologischen Material über eine möglichst große Oberfläche zu gewährleisten. Andererseits ist die Kontaktfläche groß genug und homogen und gleichmäßig genug verteilt, so dass das biologische Material sicher aufliegt, ohne zu verrutschen und so dass die Übertragung eines mechanischen Stimulus in das biologische Material möglichst homogen und gleichmäßig erfolgen kann. Geeignete Ausführungsformen des Stempels 26 bzw. der Vorrichtung 27 sind in den Figuren 6 und 7 gezeigt. Die Auflagefläche oder Kontaktfläche des Stempels 26 (siehe Fig. 6) bzw. der Vorrichtung 27 (siehe Fig. 7) wird jeweils von vier radial angeordneten, zentral miteinander verbundenen "V"-förmigen Auflageelementen 41 gebildet. Dabei wird die Kontaktfläche der einzelnen "V"-förmigen Auflageelemente 41 durch Kanäle 42 voneinander getrennt. Durch die Kanäle 42 kann Kulturmedium strömen und somit, ggf. über poröse Auflageplättchen 30a bzw. 30b, auch zur Auflageseite des biologischen Materials gelangen. Damit werden die Diffusionsstrecken in solche Bereiche möglichst klein gehalten, die nicht direkt mit dem Mediumstrom in Kontakt stehen können, während für eine möglichst große Auflagefläche gesorgt wird, über die ein mechanischer Stimulus an das biologische Material übertragen werden kann.

Um eine seitliche Verschiebung des Stempels 26 und der Vorrichtung 27 zueinander zu vermeiden, werden Stempels 26 und Vorrichtung 27 im Grundkörper 21 längsaxial geführt. Hierzu und zur Ermöglichung einer reibungsarmen Bewegung können Stempel 26 und/oder Vorrichtung 27 einen nur unwesentlich geringeren Außendurchmesser aufweisen, als der vorliegende Innendurchmesser des Grundkörpers 21.

Um eine seitliche Verschiebung des biologischen Materials 29 und der Vorrichtung 27 zueinander zu vermeiden, weist die Vorrichtung 27 Führungsmittel 40 auf, die dafür sorgen, dass Stempel 26,Vorrichtung 27 und biologisches Material 29 im Reaktorraum 23 der Bioreaktorkammer 20 aufeinander ausgerichtet vorliegen, so dass sich, bei längsaxialer Bewegung des Stempels 26 und/oder der Vorrichtung 27 aufeinander zu, das biologische Material, von Stempel 26 und Vorrichtung 27 im Wesentlichen in der vorher festgelegten Ausrichtung mechanisch bewegt oder verformt wird. Solche Führungsmittel 40 können beispielsweise stiftförmige Fortsätze, insbesondere Metallstifte, umfassen oder daraus bestehen.

In Fig. 5 wird eine weitere mögliche Bauform der Bioreaktorkammer 20 dargestellt, die eine Betrachtung des biologischen Materials mit Hilfe von Mikroskopen oder anderen bildgebenden Verfahren ermöglicht. Hierzu weist die Bioreaktorkammer 20b im Unterschied zur Bauform aus Fig. 4 einen modifizierten Stempel 31 auf, in den ein zylinderförmiges, zur Verbesserung des Nährstofftransports bevorzugt poröses Material 32 eingepresst ist. Das biologische Material 29 liegt auf einer für das jeweilige bildgebende Verfahren transparenten Scheibe 33 auf, welche in eine Hülse 34 eingeklebt und mit der Dichtung 22 c über einen Klemmring 35 dicht verbunden ist. Durch die Öffnung 36 ist das biologische Material mit dem bildgebenden Verfahren darstellbar und/oder analysierbar.

### Bioreaktor:

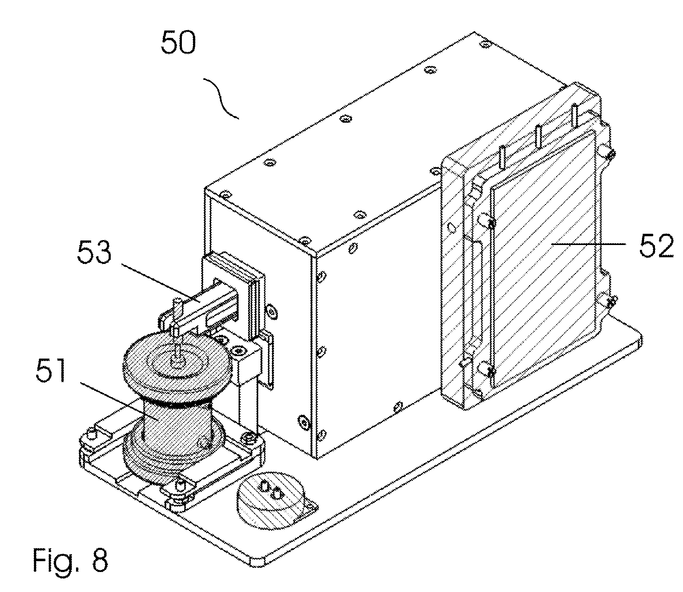

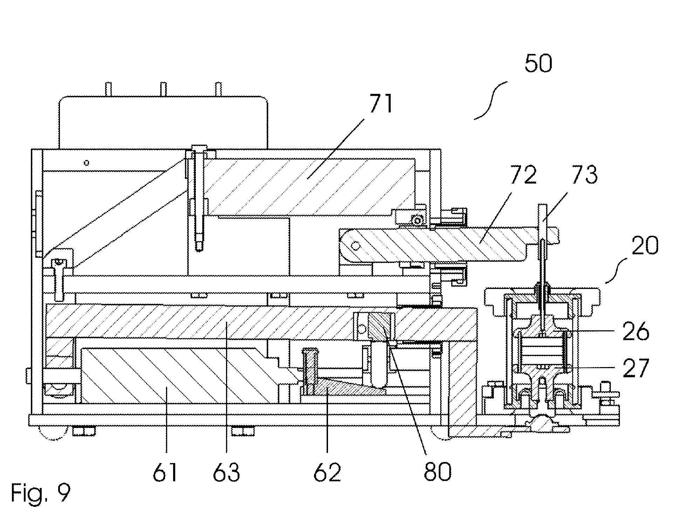

In Fig. 8 und 9 wird eine Ausführungsform des erfindungsgemäßen Bioreaktors in isometrischer bzw. in Schnittdarstellung dargestellt.

Der erfindungsgemäße Bioreaktor dient zur Kultur und mechanischen Stimulation von biologischem Material. Der in Fig. 8 gezeigte Bioreaktor 50 umfasst eine Bioreaktorkammer 51, eine Zellkultureinheit 52 und eine Einheit zur Ausübung mechanischer Stimuli 53 auf das zu kultivierende biologische Material. Bevorzugt ist der erfindungsgemäße Bioreaktor kompakt, als tragbare Einheit ausgeführt.

Dabei dient die Bioreaktorkammer 51 der Aufnahme und Kultur des biologischen Materials. Daneben ist die Bioreaktorkammer 51 auch der Ort an dem das biologische Material mechanischen Stimuli ausgesetzt werden kann. Im erfindungsgemäßen Bioreaktor 50 kann beispielsweise eine erfindungsgemäße Bioreaktorkammer eingesetzt werden (z.B. die in Fig. 3, 4 und 5 offenbarten Bioreaktorkammern 20 bzw. 20b) oder auch eine andere Bioreaktorkammer, bei der z.B. eine oder mehrere erfindungsgemäße Dichtungen (z.B. die in Fig. 1 und 2 offenbarte Dichtung 1) verwendet werden. Es können aber auch andere, herkömmliche Bioreaktorkammern zum Einsatz kommen, die die Übertragung mechanischer Stimuli in die Reaktorkammer erlauben.

Die Zellkultureinheit 52 dient dazu, die benötigten Kulturbedingungen für die Kultur des gewünschten biologischen Materials bereitzustellen und über den gewünschten Zeitraum zu gewährleisten. Dazu weist die Zellkultureinheit 52 ein Mediumreservoir, eine Vorrichtung für den Gasaustausch und eine Pumpe auf, die für die notwendige Bewegung des Kulturmediums sorgt. Darüber hinaus umfasst die Zellkultureinheit meist schlauchförmige Verbindungen zwischen den einzelnen Modulen des Zellkultursystems und der Bioreaktorkammer, so dass im Reaktorraum der Bioreaktorkammer zum gewünschten Zeitpunkt die gewünschten Kulturbedienungen bereitgestellt werden können.

In einer bevorzugten Ausführungsform kann die Pumpe als regel- und/oder steuerbare Mikropumpe ausgestaltet sein. Die Verwendung einer Mikropumpe erlaubt eine möglichst kompakte und platzsparende Gestaltung des Bioreaktors. Geeignete Mikropumpen werden beispielsweise von der Firma thinXXS Microtechnology AG, DE, hergestellt und vertrieben.
Gleichzeitig oder alternativ kann die Zellkultureinheit 52 ein Mediumreservoir mit integrierter Gasaustauschmembran, z.B. einer Silikonmembran, aufweisen. Diese Art des Gasaustauschs erlaubt ebenfalls eine möglichst kompakte, platzsparende Gestaltung des Bioreaktors. Durch den Einsatz einer integrierten Gasaustauschmembran anstatt eines herkömmlichen, direkten Gas-Flüssigkeitskontaktes, ist der Kreislauf des Kulturmediums gegenüber der Umgebung vollständig geschlossen, was das Risiko einer Infektion oder Kontamination verringert und somit längere Kulturzeiträume zulässt. Werden gleichzeitig Schlauchverbindungen mit möglichst geringer Gaspermeabilität verwendet, z.B. Schläuche aus Polyvinylchlorid (PVC) oder Polyamid (PA), so findet der Gasaustausch im Wesentlichen ausschließlich über die Gasaustauschmembran statt. Es besteht dann keine Notwendigkeit den Bioreaktor in einem CO₂-Inkubator zu betreiben. Der Bioreaktor ist somit nicht mehr an einen bestimmten Betriebsort gebunden, wie z.B. einen CO₂-Inkubator, sondern kann mobil genutzt und eingesetzt werden.

Die Einheit zur Ausübung mechanischer Stimuli 53 umfasst mindestens zwei unabhängige, getrennt voneinander regel- und/oder steuerbare Antriebe oder Aktoren. Eine mögliche Ausführungsform der Einheit zur Ausübung mechanischer Stimuli ist in Fig. 9 gezeigt.

Der erste Antrieb ist dabei als Piezoantrieb ausgestaltet. Piezoantriebe erlauben die Erzeugung und Einleitung mechanischer Kraftmuster mit hoher Dynamik und hoher Auflösung. Der Piezoantrieb erlaubt die Darstellung vielfältiger Stimulusmuster wie lineare Bewegung, einfache Wellenfunktionen (Sinus, Dreieck) oder aber auch charakteristischer Druckverläufe, wie sie typischerweise beim Gehen herrschen. Die limitierten Stellwege des Piezoantriebs 71 lassen sich durch Wahl einer geeigneten Übersetzung im gewünschten Maß erweitern. Geeignete Piezoantriebe umfassen sowohl Piezoaktoren, als auch Piezomotoren. Solche Piezoantriebe sind dem Fachmann bekannt und er hat keine Schwierigkeiten bekannte Piezoantriebe derart anzupassen, dass diese in einem erfindungsgemäßen Bioreaktor einsetzbar sind. Der Piezoantrieb ist derart ausgestaltet und positioniert, dass er in der Lage ist die Erzeugung und ggf. Einleitung mechanischer Stimuli in das biologische Material zu gewährleisten. Bevorzugt werden Piezoantriebe eingesetzt, die die Erzeugung und ggf. Einleitung mechanischer Stimuli in das biologische Material mit einer maximalen Frequenz von ≥ 5 Hz gewährleisten, besonders bevorzugt mit einer maximalen Frequenz von ≥ 10Hz, ganz besonders bevorzugt mit einer maximalen Frequenz von ≤ 100 Hz.
Der zweite Antrieb ist ein Linearantrieb 61. Der Linearantrieb 61 erlaubt eine Regulation der Position des biologischen Materials im Reaktorraum der Bioreaktorkammer entlang der Längsachse der Bioreaktorkammer. Bevorzugt ist der Linearantrieb 61 als elektromechanischer Linearantrieb ausgestaltet.

Der Linearantrieb 61 des Bioreaktors 50, so wie er in Fig. 9 dargestellt ist, ist im Gehäuse des Bioreaktors 50 angeordnet. Über eine Übersetzung 62 werden die horizontalen Bewegungen des Linearantriebs 61 in im Wesentlichen vertikale Bewegungen eines Auslegers 63 entlang der Längsachse der Bioreaktorkammer übersetzt. Der Ausleger 63 steht in Kontakt mit der Vorrichtung 27 zur Aufnahme von biologischem Material der Bioreaktorkammer 20. Dabei werden die mechanischen Stimuli vom Ausleger 63 mittels der beweglichen Vorrichtung 27 auf das biologische Material übertragen. In einer besonderen Ausführungsform kann der Teil des Auslegers 63, der mit der Hülse 34 in Kontakt steht sowie der Boden der Bioreaktorkammer 20b derart ausgeführt sein, dass das biologische Material durch den Boden der Bioreaktorkammer 20b mikroskopierbar ist.

Der Piezoantrieb 71 des Bioreaktors 50, wie er in Fig. 9 dargestellt ist, ist ebenfalls im Gehäuse des Bioreaktors 50 angeordnet. Über einen, an einem Gelenk fixierten Auflagearm 72 werden die mechanischen Bewegungen des Piezoantriebs 71 von maximal 500 µm auf einen Stellweg von ca. 1500 µm übersetzt. Der Auflagearm 72 weist an seinem, dem Piezoantrieb 71 abgewandten Ende eine bevorzugt mit einfachen Mitteln zu befestigende und zu lösende Übersetzung 73 auf, die eine Übersetzung der Bewegungen des Auflagearms 72 in eine Bewegung des Stempels 26 entlang der Längsachse der Bioreaktorkammer 20 erlaubt. Bevorzugt erlaubt die Übersetzung 73 ausreichend Spiel, so dass die radiale Bewegung des Auflagearms 72 um das Gelenk übersetzt wird, in eine im Wesentlichen lineare Bewegung, die möglichst exakt der Längsachse der Bioreaktorkammer 20 folgt, so dass, auch während der Übertragung von mechanischen Stimuli auf das biologische Material, die Auflageflächen des Stempels 26 und der Vorrichtung 27 möglichst parallel zueinander bleiben.

Der Bioreaktor 50 kann zusätzlich einen Kraftsensor 80 aufweisen, der derart angeordnet ist, dass darüber die tatsächlich auf das biologische Material einwirkenden Kräfte bestimmbar sind. Dazu kann der Kraftsensor mit dem Ausleger 63 des Linearantriebs verbunden sein. Dadurch ist es möglich, direkt die Kräfte zu messen, die tatsächlich auf das biologische Material einwirken. Es ist bekannt, dass sich biologisches Material über die Zeit durch die Einwirkung mechanischer Kräfte dauerhaft verformen kann. So kann es durch wiederholte oder lang anhaltende, gerichtete Kompression des biologischen Materials zu einer dauerhaften Abnahme der Dicke des biologischen Materials entlang der Richtung der mechanischen Kraft kommen. Wird nun der mechanische Stimulus unverändert angeboten, so verändert sich die tatsächlich am biologischen Material wirkende Kraft über die Zeit. Der tatsächlich auf das biologische Material wirkende mechanische Stimulus nimmt über die Zeit ab. Über den Kraftsensor 80 kann diese Abnahme bestimmt werden. Wird eine solche Abnahme festgestellt, so kann mittels des Linearantriebs 61 die Position des biologischen Materials im Reaktorraum der Bioreaktorkammer 20 derart nachgestellt werden, dass das biologische Material, bei konstantem mechanischem Stimulus des Piezoantriebs 71, wieder solchen Kräften ausgesetzt ist, die den ursprünglich auf das biologische Material einwirkenden Kräften im Wesentlichen entsprechen. Eine Materialverformung des biologischen Materials über die Zeit kann somit ausgeglichen werden, ohne dass der Stellweg des Piezoantriebs selbst nachgeregelt werden muss.

Die Messung der tatsächlich auf das biologische Material einwirkenden Kräfte mit Hilfe des Kraftsensors 80 ermöglicht in Kombination mit dem hochauflösenden Piezoantrieb 71 außerdem die Bestimmung von Materialkenngrößen wie beispielsweise dem Elastizitätsmodul des biologischen Materials oder von zellbeladenen aber auch zellfreien Biomaterialien oder anderen Werkstoffen wie beispielsweise Polymermaterialien bei gleichzeitiger Kultivierung im Bioreaktor.

Der erfindungsgemäße Bioreaktor kann zusätzlich eine Steuer- und/oder Regelungseinheit aufweisen. Diese Steuer- und/oder Regelungseinheit ist derart ausgestaltet und angeordnet, dass die Position des biologischen Materials im Reaktorraum der Bioreaktorkammer in Abhängigkeit von den am Kraftmesser gemessenen Kraftwerten veränderbar ist. Die Steuer- und/oder Regelungseinheit ist in der Lage, Messwerte des Kraftmessers aufzunehmen und zu verarbeiten. Die gemessenen Kraftwerte werden dann mit einem Sollwert verglichen und es wird ggf. eine Abweichung bestimmt. Überschreitet die Abweichung einen vorher festgelegten Schwellenwert, so kann mittels der Steuer- und/oder Regelungseinheit der Linearantrieb derart angesteuert werden, dass über den Linearantrieb die Position des biologischen Materials in der Bioreaktorkammer derart verändert wird, dass die am Kraftmesser gemessenen Kraftwerte nicht mehr zu Abweichungen führen, die den vorher festgelegten Schwellenwert überschreiten.

Zusätzlich kann die Steuer- und/oder Regelungseinheit dazu ausgelegt sein, noch weitere Steuer- und/oder Regelungsaufgaben im erfindungsgemäßen Bioreaktor zu übernehmen. Über die Steuer- und/oder Regelungseinheit kann es beispielsweise möglich sein auch die Zellkultureinheit zu bedienen und/oder durch die Bedienung des Piezoantriebs die mechanischen Stimuli zu bestimmen, die auf das biologische Material einwirken sollen.

### Weitere Angaben zur Erfindung:

Die Motivation für die vorliegende Erfindung ist die Bereitstellung eines Test-Gerätes, welches das biochemische und mechanische Umfeld des Frakturspalts simuliert.

Die Erfahrung mit vorhandenen Bioreaktoren hat den Fokus auf die Entwicklung eines geschlossenen Systems für lange Kultivierungszeiten (> 1 Woche) und definierte, variierbare Kulturbedingungen gerichtet. Ein zentraler Punkt ist die Möglichkeit, variable zyklische Kraftmuster in einem breiten Frequenzbereich realisieren zu können, sowie eine automatische Proben-Neupositionierung, um die irreversible Probendeformation während einer wiederkehrenden Kompression auszugleichen.

Der Bioreaktor ist bevorzugt ein geschlossenes System, bestehend aus einer Zellkultur- Einheit (Flüssigkeitskreislauf und seine Komponenten) und einer Einheit zur Erzeugung der mechanischen Kraftmuster. Die Schnittstelle zwischen den beiden Einheiten ist die Reaktorkammer.

Neben der Reaktorkammer enthält die Zellkultur-Einheit ein Reservoir mit einer Silikonmembran für den Gasaustausch sowie eine Mikropumpe. Daran angeschlossen ist eine Gasmischeinheit, um die gewünschte Sauerstoff-Konzentration im Kreislauf zu erzielen und den pH-Wert auf einem konstanten Niveau zu halten. So ist es möglich, variable Sauerstoff-Partialdrücke zu realisieren und somit z. B. ein hypoxisches Umfeld zu erzeugen, wie es bekanntermaßen in dem frühen Stadium der Frakturkallusentwicklung auftritt. An das Reservoir optional angebrachte optochemische und elektrische Sensoren messen die wichtigsten Zellkulturparameter (pO2, pH, Temperatur). Das System kann für ein geringes Gesamtvolumen an Zellkulturmedium ausgelegt sein (15-25ml), um Proteine, die in das Medium sezerniert werden, sensitiver detektieren und die Kosten bei der Applikation von Wachstumsfaktoren gering halten zu können. Jederzeit können kleine Mengen Medium für eine Analyse entnommen werden. Das Medium kann darüber hinaus bei längerer Kultivierungsdauer komplett ausgetauscht werden. Durch den Einsatz einer GasaustauschMembran anstatt eines direkten Gas-/Flüssigkeits-Kontaktes ist der Kreislauf des Zellkulturmediums gegenüber der Umgebung vollständig geschlossen, was das Risiko einer Infektion minimiert. Durch die Verwendung einer Mikropumpe, deren Anwendung in der Zellkultur bisher nicht bekannt ist, in Kombination mit Schlauchleitungen geringer Gaspermeabilität (z. B. PVC, PA) findet der Gasaustausch fast ausschließlich über die Gasaustauschmembran, z.B. als Silikonmembran, statt. Folglich besteht keine Notwendigkeit für die Verwendung eines CO₂-Inkubators. In diesem Kreislauf sollte es auch möglich sein, den Sauerstoff-Verbrauch des biologischen Materials als Indikator für die Proliferation oder Differenzierung zu messen.

In der Einheit zur Erzeugung der mechanischen Kraftmuster wird ein Piezoaktor mit hoher Dynamik und hoher Auflösung eingesetzt. Hierdurch können vielfältige Muster wie einfache Wellen-Funktionen (Sinus, Dreieck etc) oder aber charakteristische Druckverläufe erzeugt werden, die aus der Messung physiologischer Kräfte (z. B. beim Gehen) mit Hilfe instrumentierter Implantate (Gruppe von Prof. Bergman im Julius-Wolff-Institut) abgeleitet werden. Abhängig von der Belastung und dem Stellweg können Frequenzen bis ca. 100 Hz realisiert werden. Da der Stellweg des Piezoaktors auf 500 µm limitiert ist (im Bioreaktor werden durch eine Hebel-Übersetzung 1500 µm erreicht), wird ein hochauflösender Linearantrieb verwendet, um das biologische Material im Arbeitsbereich des Piezoaktors zu positionieren. Ein hochdynamischer Piezoantrieb und ein langsamer Linearantrieb mit großem Stellweg werden folglich zu einer sehr kompakten Einheit kombiniert, welche die gewünschten Bewegungsmuster realisieren kann. Die auf das biologische Material applizierte Kraft kann durch eine Kraftmesszelle erfasst werden, die mit dem Auflagearm verbunden ist. Dieser Arm wird durch den Linearantrieb bewegt. Da keine störenden Beschleunigungs- oder Reibungskräfte auftreten, kann die Kraft, die auf die Probe wirkt, sehr präzise gemessen werden. Der Linearantrieb wird zusätzlich benutzt, um automatisch die Probenposition nachzuregeln. Dies ist notwendig da die Probendicke durch die Kompression im Laufe der Zeit abnimmt, wie sich in Versuchsreihen in unserem bestehenden Bioreaktor-System gezeigt hat.

Die zylinderförmige Probe befindet sich im Zentrum der Reaktorkammer. Hier wird sie mit Zellkulturmedium versorgt, während sie zyklisch durch die über zwei Druckstempel wirkende Kraft axial komprimiert wird. Das Übertragen der Bewegung auf die in der Kammer befindlichen Stempel wird durch zwei speziell geformte Dichtungen ermöglicht. Die Dichtungen bestehen aus Silikon, weisen einen geringen Widerstand gegenüber den applizierten Kräften auf und wirken gleichzeitig als Abdichtung für die Reaktorkammer. Die Form der Druckstempel kann variiert werden, um verschiedene Arten von Medienflüssen wie eine freie Umströmung der Probe oder eine erzwungene Perfusion zu realisieren. Über Aussparungen in den Stempeln steht die Probe auch an den Stirnseiten in Kontakt mit dem Medium. Eine leicht modifizierte Kammer ermöglicht einen optischen Zugang zur Probe. So kann beispielsweise ein konfokales Laser-Scanning-Mikroskop zur Untersuchung der Probe eingesetzt werden. Das Imaging kann durchgeführt werden ohne den Fluss des Zellkulturmediums zu unterbrechen, ebenso während der Kompression der Probe oder ohne die Wirkung äußerer Kräfte.

Die innovativen Kernpunkte des Bioreaktors sind im Folgenden aufgelistet und erläutert:
- Definierte biochemische und mechanische Umgebung, welche die physiologische Situation im Frakturspalt simuliert. Dies wird in vollständig unabhängigen Einheiten realisiert. Durch die Nutzung getrennter Zellkultur-Kreisläufe kann die Genexpression jeder Probe als wichtiger biochemischer Indikator für die Zelldifferenzierung analysiert werden. Durch den Einsatz separater mechanischer Einheiten für jede Probe kann sichergestellt werden, dass die gewünschten definierten Kraftmuster realisiert werden.
- Individuelle und automatische Anpassung an die Veränderungen der Probendicke. Die genaue Messung der auf die Probe wirkenden Kraft ermöglicht es, die Abnahme der Probenstärke zu erfassen. Hierzu wird eine Analysefunktion auf das Kraftsignal angewendet die das Abheben des Stempels von der Probenoberfläche erkennt. Die Probenposition wird dann automatisch angepasst (angehoben), um die gewünschten Bedingungen (z. B. konstante Maximalwerte von Kraft oder Kompression) zu realisieren.
- Geschlossenes System: Der Bioreaktor ist ein geschlossenes System nicht nur in Bezug auf die Flüssigkeit (Minimierung des Infektionsrisikos), sondern auch in Bezug auf die im Zellkulturmedium gelösten Gase (Schläuche, Pumpe). In Kombination mit einem beheizbaren Gehäuse sind die individuellen Bioreaktor-Einheiten vollkommen unabhängig von einem Inkubator.
- Kompaktes System: Die Kombination eines Piezoaktors mit einem präzisen Linearantrieb in der mechanischen Einheit ist Voraussetzung für das kompakte Design des Bioreaktors. Bei einer Größe entsprechend eines 1 Liter Tetrapacks sind alle wesentlichen Komponenten enthalten (Reaktorkammer-, Gasaustausch-Reservoir, Pumpe, Motor für die Kraft-Applikation, Kraftmesszelle). Die Bioreaktor-Einheiten sind einfach zu handhaben, können komplett in einer Clean Bench platziert und als Gesamtsystem zu einem Analyse-Gerät wie einem Mikroskop überführt werden.
- Optischer Anschluss: Eine speziell konstruierte Reaktorkammer bietet die Möglichkeit die Probe mit einem konfokalen Laser-Scanning-Mikroskop oder anderen optischen Instrumenten zu betrachten.

### Mögliche Verwendungen der Erfindungsgegenstände umfassen:

Die Verwendung des erfindungsgemäßen Bioreaktors für die Entwicklung und/oder Testung von Arzneimitteln, insbesondere von Arzneimitteln zur Behandlung von Komplikationen in der Knochenheilung. Solche Arzneimittel können in der Endoprothetik (Knie, Hüfte, Schulter), für operative Zahnheilkunde oder in der plastischen Chirurgie Anwendung finden. Untersuchungen in dem erfindungsgemäßen Bioreaktor können helfen, die Anzahl der notwendigen Tierversuche zu reduzieren.

Die Verwendung des erfindungsgemäßen Bioreaktors in der Testung von Biomaterialien beispielsweise bezüglich des Einfluss dieser Materialien auf die Zellentwicklung, bezüglich der zeitabhängigen Veränderung der mechanischen Eigenschaften dieser Materialien, bezüglich der Materialdegradation oder -resorption oder bezüglich der Freisetzung von Wirkstoffen aus hiermit beladenen Materialien. Diese Untersuchungen können z.B. einen Hinweis darauf geben, welchen Effekt die Materialien auf die heilende Fraktur haben.

Der erfindungsgemäße Bioreaktor kann verwendet werden, um eine optimierte Zellzusammensetzung für die lokale Zelltherapie bei Knochenfrakturen und -defekten zu finden.

## Patentansprüche

1. Bioreaktor (50) zur Kultur und mechanischen Stimulation von biologischem Material umfassend eine Bioreaktorkammer, eine Zellkultureinheit (52) und eine Einheit (53) zur Ausübung mechanischer Stimuli auf das zu kultivierende biologische Material, **dadurch gekennzeichnet, dass** die Einheit (53) zur Ausübung mechanischer Stimuli mindestens zwei unabhängige, regel- und/oder steuerbare Antriebe aufweist, wobei ein erster Antrieb ein Piezoantrieb (71) ist, der eine Erzeugung mechanischer Stimuli erlaubt, und ein zweiter Antrieb ein Linearantrieb (61) ist, der eine Regulation der Position des biologischen Materials in der Bioreaktorkammer erlaubt.

2. Bioreaktor (50) nach Anspruch 1, **dadurch gekennzeichnet, dass** der Piezoantrieb (71) die Erzeugung mechanischer Stimuli mit einer maximalen Frequenz von ≥ 5 Hz erlaubt.

3. Bioreaktor (50) nach einem der Ansprüche 1 oder 2, **dadurch gekennzeichnet, dass** der Bioreaktor (50) zusätzlich einen Kraftsensor (80) aufweist, der derart angeordnet und ausgestaltet ist, dass darüber die tatsächlich auf das biologische Material einwirkenden Kräfte bestimmbar sind.

4. Bioreaktor (50) nach Anspruch 3, **dadurch gekennzeichnet, dass** der Bioreaktor (50) zusätzlich eine Steuer- und/oder Regelungseinheit aufweist, die derart angeordnet und ausgestaltet ist, dass die Position des biologischen Materials in der Bioreaktorkammer in Abhängigkeit von den am Kraftmesser gemessenen Werten regulierbar ist

5. Dichtung (1) zur Abdichtung einer Bioreaktorkammer, umfassend:
a) ein Formelement (2) ausgebildet zur flüssigkeits- und/oder gasdichten Abdichtung eines Kontaktbereichs der Dichtung (1) mit einer Oberfläche einer Bioreaktorkammer; und
b) ein Dichtungselement (3), wobei das Dichtungselement eine Rollmembran aufweist, die einen Aufnahmebereich (4) zur flüssigkeits- und/oder gasdichten Aufnahme einer Kolbenstange und einen Rollbereich (5) umfasst.

6. Dichtung (1) nach Anspruch 5, **dadurch gekennzeichnet, dass** im Aufnahmebereich (4) eine Kolbenstange derart kraftschlüssig, stoffschlüssig und/oder formschlüssig aufnehmbar ist, dass eine Relativbewegung von Kolbenstange zum Aufnahmebereich (4) im Wesentlichen unterbunden ist.

7. Dichtung (1) nach einem der Ansprüche 5 oder 6, **dadurch gekennzeichnet, dass** der Rollbereich (5) derart ausgestaltet ist, dass der Aufnahmebereich (4) in längsaxialer Richtung über eine vorher festgelegte Strecke bewegbar ist, ohne dass es im Wesentlichen zu einer Verformung des Dichtungselementes (3) außerhalb der Rollmembran kommt.

8. Dichtung (1) nach einem der Ansprüche 5 bis 7, **dadurch gekennzeichnet, dass** die Dichtung (1) Silikon oder ein Silikon-haltiges Material aufweist oder daraus besteht.

9. Dichtung (1) nach einem der Ansprüche 5 bis 8, **dadurch gekennzeichnet, dass** das Formelement (2) ausgebildet zur flüssigkeits- und/oder gasdichten Abdichtung eines Kontaktbereichs der Dichtung (1) mit einer Oberfläche der Bioreaktorkammer eine Dichtkante und/oder eine Dichtfläche aufweist und gegebenenfalls eine oder mehrere Dichtwülste umfasst.

10. Bioreaktorkammer (20, 20b) zur Verwendung in einem Bioreaktor, umfassend einen Grundkörper (21) und mindestens eine Dichtung (1, 22a, 22b, 22c) nach einem der Ansprüche 1 bis 5, wobei:
- Grundkörper (21) und Dichtung (1, 22a, 22b, 22c) zusammen einen flüssigkeits - und/oder gasdichten Reaktorraum (23) bilden;
- die Bioreaktorkammer (20, 20b) wenigstens zwei Anschlüsse (25a, 25b) aufweist, über die dem Reaktorraum (23) Flüssigkeit und/oder Gas zu- und/oder abführbar sind;
- im Reaktorraum (23) ein Stempel (26, 31) angeordnet ist, der über eine, in der Aufnahme des Dichtungselements der Dichtung (1, 22a, 22b) gelagerte Kolbenstange in längsaxialer Richtung bewegbar ist; und
- im Reaktorraum (23) eine Vorrichtung (27, 34) zur Aufnahme von biologischem Material derart angeordnet ist, dass auf der Vorrichtung (27, 34) platziertes biologisches Material durch längsaxiale Bewegung des Stempels (26, 31) und/oder der Vorrichtung (27, 34) einer mechanischen Kraft aussetztbar ist.

11. Bioreaktorkammer (20, 20b) nach Anspruch 10, **dadurch gekennzeichnet, dass** der oder die Stempel (26, 31) und/oder die Vorrichtung (27, 34) zur Aufnahme von biologischem Material derart ausgestaltet sind, dass platziertes biologisches Material während der Kultur von Kulturmedium umströmbar ist.

12. Bioreaktorkammer (20, 20b) nach Anspruch 11, **dadurch gekennzeichnet, dass** der oder die Stempel (26, 31) und/oder die Vorrichtung (27, 34) zur Aufnahme von biologischem Material einen Bereich aufweisen, der während der Kultur mit dem biologischen Material direkt oder indirekt in Kontakt treten kann, wobei dieser Bereich von vier radial angeordneten, zentral miteinander verbundenen V-förmigen Auflageelementen (41) gebildet wird und die Kontaktflächen der einzelnen V-förmigen Auflageelemente (41) durch mit Kulturmedium durchströmbare Kanäle (42) voneinander getrennt vorliegen.

13. Bioreaktorkammer (20, 20b) nach einem der Ansprüche 10 bis 12, **dadurch gekennzeichnet, dass** die Bioreaktorkammer (20, 20b) zwei gegenüberliegend angeordnete Dichtungen (1, 22a, 22b) nach einem der Ansprüche 5 bis 9 aufweist, und im Reaktorraum (23) zwei Stempel (26, 31) sich gegenüberstehend angeordnet sind, wobei die Stempel (26, 31) unabhängig voneinander über die in der Aufnahme der jeweiligen Dichtung (1, 22a, 22b) gelagerte Kolbenstange in längsaxialer Richtung bewegbar sind und wobei einer der beiden Stempel (26, 31) als Vorrichtung (27, 34) zur Aufnahme von biologischem Material dient.

14. Bioreaktorkammer (20, 20b) nach Anspruch 13, **dadurch gekennzeichnet, dass** das biologische Material (29) durch Führungsmittel (40) auf der Vorrichtung (27) zentriert vorliegen, so dass sich, bei längsaxialer Bewegung des Stempels (26, 31), das biologische Material von Stempel (26, 31) und Vorrichtung (27, 34) im Wesentlichen in der vorher festgelegten Ausrichtung mechanisch bewegt oder verformt wird.

15. Bioreaktor (50) nach einem der Ansprüche 1 bis 4, **dadurch gekennzeichnet, dass** die Bioreaktorkammer eine Bioreaktorkammer (20) nach einem der Ansprüche 10 bis 14 ist und/oder die Bioreaktorkammer eine oder mehrere Dichtungen (1, 22a, 22b) nach einem der Ansprüche 5 bis 9 aufweist.
